# EUROPEAN PATENT SPECIFICATION

(11) **EP 4 238 527 B1**
(45) Date of publication and mention of the grant of the patent: **01.10.2025**
(21) Application number: 21886503.8
(22) Date of filing: 11.08.2021
(51) Int. Cl.: A61C 5/77, A61C 9/00, A61C 13/00, A61C 13/08, A61C 13/20

(54) **PREFABRICATED CROWNS FOR DENTAL PROSTHETIC RESTORATION AND MANUFACTURING METHOD**
VORGEFERTIGTE KRONEN FÜR ZAHNPROTHETISCHE RESTAURATIONEN UND VERFAHREN ZU IHRER HERSTELLUNG
COURONNES PRÉFABRIQUÉES POUR RESTAURATION DENTAIRE PROTHÉTIQUE ET PROCÉDÉ DE FABRICATION

(30) Priority: 27.10.2020 KR 20200140257
(43) Date of publication of application: 06.09.2023
(73) Proprietor: Hass Corporation, Gangneung-si, Gangwon-do, 25452 (KR)
(72) Inventor: KIM, Yong-su, Gangneung-si Gangwon-do 25497 (KR); JEON, Hyun-jun, Busan 47508 (KR); OH, Kyung-sik, Incheon 22003 (KR); HONG, Young-pyo, Gangneung-si, Gangwon-do 25598 (KR); LIM, Hyung-bong, Ansan-si, Gyeonggi-do 15521 (KR); HAM, Duck-won, Gangneung-si, Gangwon-do 25514 (KR); KOH, Hwan-soon, Anyang-si, Gyeonggi-do 13910 (KR)
(74) Representative: Gulde & Partner
(86) International application number: PCT/KR2021/010662
(87) International publication number: WO 2022/092504

(56) References cited:
- WO-A2-2005/007007
- JP-A- 2010 503 437
- JP-B2- 6 741 592
- KR-A- 20020 065 882
- KR-A- 20110 112 740
- KR-B1- 101 071 554
- KR-B1- 102 117 441
- KR-B1- 102 117 441
- KR-B1- 102 286 271
- US-A1- 2009 317 780
- US-A1- 2010 049 351
- US-A1- 2016 291 585
- US-A1- 2020 172 444

## Description

### [Technical Field]

The present invention relates to a prefabricated crown for dental prosthetic restoration and a method for manufacturing the same, and more specifically, to a technology cable of quickly providing a prefabricated crown to patients when necessary by tabulating the variables of the type, three-dimensional dimension, color and material of prefabricated crowns for sub-gingival and supra-gingival and actual and try-in crowns, and manufacturing a prefabricated crown corresponding to the table in advance.

### [Background Art]

In general, single-tooth prosthetic restoration refers to a procedure for restoring teeth severely damaged by dental caries or trauma with a crown. In addition, the crown used in prosthetic restoration is divided into a patient customized crown, in which a crown suitable for an individual patient is directly fabricated and provided, and a prefabricated crown, in which a crown is prefabricated and provided to the patient when necessary.

Patient customized crowns are fabricated through a complex fabrication process in a factory or dental clinic, and there is a disadvantage in that the fabrication process is lengthened due to repair or failure during fabrication, and accordingly, the clinical process is lengthened at the same time, and additional fabrication processes are required, so fabrication costs and medical expenses are excessively generated.

In particular, since it is fabricated with a patient customized crown, it takes a long time to fabricate, so there is a problem in that it is difficult to immediately perform prosthetic restoration treatment on a patient.

Moreover, although interest in patient customization and aesthetic improvement with high medical costs is increasing as economic power improves in recent years, the low-income class has no choice but to maintain the oral environment in a state of permanently missing teeth by either not receiving timely treatment or giving up treatment because it is difficult to receive treatment due to expensive patient customized crowns.

In addition, the prefabricated crown means an off-the-shelf in a finished product state for the purpose of crown treatment, preferably a dental restoration material provided in a finished product state having an anatomically designed prefabricated shape capable of immediate prosthetic restoration of a single tooth.

Such a prefabricated crown is cheaper than a patient customized crown, but there is a problem in that it does not reach a comparable level in strength and aesthetics. Examples may be found in WO 2005/007007 A2, US 2016/0291585 A1, KR 102 117 441 B1, US 2009/317780 A1 or US 2020/172444 A1.

### [Disclosure]

### [Technical Problem]

The present invention has been proposed to solve the above problems, and it is an object of the present invention to provide a method for manufacturing a prefabricated crown capable of providing prosthetic restoration to patients quickly when necessary by tabulating the variables of the type, three-dimensional dimension, color and material of actual crowns for sub-gingival/supra-gingival and try-in crowns for sub-gingival/supra-gingival, and manufacturing a prefabricated crown corresponding to the table in advance, and a prefabricated crown manufactured therefrom.

### [Technical Solution]

The invention is defined by the appended claims.

In order to solve the above object, an embodiment of the present invention provides a method for manufacturing a plurality of prefabricated crowns, including try-in and actual crowns.

The method is a method for manufacturing a plurality of prefabricated crowns, including try-in and actual crowns, by processing and tabulating the prefabricated crowns for sub-gingival and supra-gingival and
the actual and try-in crowns by type, size, material and color by a table system 1, and comprises:
a first step S100 of classifying a plurality of prefabricated crowns by type and size by a first module 5 of the table system 1;
a second step S110 of classifying the plurality of prefabricated crowns by color and material by a second module 7 of the table system 1;
a third step S120 of tabulating the type, size, color and material data of the prefabricated crowns classified in the first and second steps S100, S110 by a third module 9 of the table system 1, and assigning an identification code to each data;
a fourth step S130 of manufacturing a plurality of prefabricated crowns including the try-in and actual crowns corresponding to the table system; and
a fifth step S140 of inputting the measured type, size, color and material data of patient's tooth into a fourth module 11 of the table system 1 to select a prefabricated crown corresponding there to,
wherein the prefabricated crown has three-dimensional dimensions (X, Y, Z) meaning the length in the X-axis, Y-axis and Z-axis directions, and
the three-dimensional dimensions of the try-in crown are smaller than the three-dimensional dimensions of the actual crown, and
the three-dimensional dimensions of the try-in crown are smaller by -1.5% to -0.3% based on the three-dimensional dimensions of the actual crown.

In a specific embodiment of the present invention, in the fourth step S130, the prefabricated crowns are manufactured by any one of an injection molding method, a subtractive manufacturing method, and an additive manufacturing method, and
the injection molding method may comprise molding a ceramic composition comprising a zirconium oxide stabilized with yttrium oxide or a zirconium oxide containing a colorant stabilized with yttrium oxide, and the steps of:
mixing 70 to 85% by weight of ceramic and 15 to 30% by weight of at least one polymer selected from the group consisting of paraffin wax, low-density polyethylene and ethylene vinyl acetate by heating at 150 to 180°C;
injection molding the heated and mixed mixture at a temperature of 150 to 200°C and a pressure of 30 to 40 MPa for 30 to 100 seconds to manufacturing a injection-molded body of a prefabricated crown;
debinding the injection molded body by a first heat treatment at 25 to 250°C for 20 to 30 hours, followed by a second heat treatment at 200 to 550°C for 20 to 30 hours;
sintering the debinded injection molded body by heat treatment at 1,400 to 1,600°C for 1 to 5 hours; and
polishing the outer surface of the heat-treated sintered body to impart gloss.

In another specific embodiment of the present invention, in the fourth step S130, the prefabricated crowns are manufactured by any one of an injection molding method, a subtractive manufacturing method, and an additive manufacturing method, and
the subtractive manufacturing method comprises subtractive manufacturing a ceramic blank comprising a zirconium oxide stabilized with yttrium oxide and/or a zirconium oxide containing a colorant stabilized with yttrium oxide, and the steps of:
clamping a ceramic blank comprising a zirconium oxide stabilized with yttrium oxide and/or a zirconium oxide containing a colorant stabilized with yttrium oxide to a subtractive manufacturing machine;
importing a file of an anatomically designed prefabricated shape for each single tooth into the subtractive manufacturing machine to subtractive manufacture the clamped blank into a prefabricated crown, and processing it into a workpiece of a prefabricated crown;
debinding the workpiece by a first heat treatment at 25 to 600°C for 1 to 10 hours, followed by a second heat treatment at 600 to 1000°C for 1 to 10 hours;
sintering the debinded workpiece by heat treatment at 1,500 to 1,600°C for 1 to 5 hours; and
polishing the outer surface of the heat-treated sintered body to impart gloss, and wherein the blank may have a disk shape and may be processed into a plurality of crown workpieces as a blank for single tooth.

In another specific embodiment of the present invention, in the fourth step S130, the prefabricated crowns are manufactured by one of an injection molding method, a subtractive manufacturing method, and an additive manufacturing method, and
the additive manufacturing method comprises additive manufacturing a ceramic composition comprising a zirconium oxide stabilized with yttrium oxide or a zirconium oxide containing a colorant stabilized with yttrium oxide, and the steps of:
injecting the ceramic composition into the additive manufacturing machine;
importing a file of an anatomically designed prefabricated shape for each single tooth into the additive manufacturing machine to additive manufacture the injected composition into a prefabricated crown, and forming it into a work piece of a prefabricated crown;
debinding the work piece by a first heat treatment at 25 to 600°C for 1 to 10 hours, followed by a second heat treatment at 600 to 1000°C for 1 to 10 hours;
sintering the debinded work piece by heat treatment at 1500 to 1,600°C for 1 to 5 hours; and
polishing the outer surface of the heat-treated sintered work piece to impart gloss, and
wherein the ceramic composition is made by mixing a thermosetting resin comprising 5 to 10% by weight of an epoxy resin, 1 to 10% by weight of maleic acid, 1 to 10% by weight of 3,6,9-trioxadecanoic acid and 1 to 10% by weight of an amine compound based on 100% by weight of a liquid silicone resin, and modified zirconium oxide powders stabilized with yttrium oxide or zirconium oxide powders containing a colorant stabilized with yttrium oxide in a weight ratio of 0.8 to 1.2:1, wherein the modified zirconium oxide powders stabilized with yttrium oxide or zirconium oxide powders containing a colorant stabilized with yttrium oxide is obtained by mixing a composition comprising 5 to 15% by weight of a silane compound, 1 to 10% by weight of an ethyl acrylate monomer and 1 to 10% by weight of a solvent at a temperature of 120 to 250°C based on 100% by weight of unmodified zirconium oxide powder stabilized with yttrium oxide or zirconium oxide powder containing a colorant stabilized with yttrium oxide, and wherein the work piece may be formed from a single tooth work piece into a plurality of crown formed bodies.

In one embodiment of the present invention, the first step S100 may be performed in a method of classifying the prefabricated crowns by type and size by the first module 5 by inputting whether the prefabricated crowns are sub-gingival or supra-gingival, and actual or try-in through the input part 3 of the table system 1 and inputting the three-dimensional size.

In one embodiment of the present invention, the second step S110 may be performed in a method of inputting the material and color of the prefabricated crowns through the input part 3 of the table system 1 and classifying the prefabricated crowns by material and color by the second module 7.

In one embodiment of the present invention, the third step S120 comprises matching and tabulating the type, size, color and material data of the prefabricated crowns classified by the first module 5 and the second module 7 with each other by the third module 9, and
may be performed in a method of tabulating each identification code by assigning identification codes by classifying the types of the prefabricated crowns into supra-gingival or sub-gingival, or actual or try-in; assigning identification codes by aligning the sizes of the prefabricated crowns so that each prefabricated crown may be distinguished from each other; assigning identification codes so that a plurality of ceramics may be distinguished from each other in the materials of the prefabricated crowns and expressing the content of the ceramics to be distinguished in the form of numbers in the identification codes; and assigning identification codes so that the colorants that may include a plurality of fluorescent materials may be distinguished from each other in the colors of the prefabricated crowns and expressing the content of the colorants to be distinguished in the form of numbers in the identification codes. In one embodiment of the present invention, a plurality of prefabricated crowns comprising a try-in crown and an actual crown prepared from the above method are provided.

In a specific embodiment of the present invention, the prefabricated crown comprises a sub-gingival crown and a supra-gingival crown.

In the present invention, the prefabricated crown has three-dimensional dimensions (X, Y, Z) meaning the length in the X-axis, Y-axis and Z-axis directions, and the three-dimensional dimensions of the try-in crown are smaller than the three-dimensions dimension of the actual crown and the three-dimensional dimensions of the try-in crown are smaller by -1.5% to -0.3% based on the three-dimensional dimensions of the actual crown.

In a specific embodiment of the present invention, the prefabricated crown comprises subtractive manufacturing a zirconium oxide ceramic stabilized with yttrium oxide or a zirconium oxide ceramic containing a colorant stabilized with yttrium oxide.

In a specific embodiment of the present invention, the colorant comprises one or more selected from the group consisting of erbium (Er₂O₃), terbium oxide (Tb₄O₇), thulium oxide (Tm₂O₃), red iron oxide (Fe₂O₃), yellow iron oxide (Fe₂O₃), molybdenum oxide (MnO₃), manganese dioxide (MnO₂), cerium oxide (CeO₂), barium oxide (BaO), vanadium pentoxide (V₂O₅), vanadium trioxide (V₂O₃) and cobalt oxide (CO₃O₄).

In a specific embodiment of the present invention, the colorant is included in an amount of not more than 0.5% by weight based on the total weight of the zirconium oxide ceramic containing a colorant stabilized with yttrium oxide.

### [Advantageous Effects]

The present description as described above has the following effects.

First, the prefabricated crown is pre-manufactured as a prefabricated crown in a finished product state, unlike the conventional patient customized crown - a crown in which semi-finished materials are processed into finished products, and is supplied to the dental clinic as an off-the-shelf before the initial treatment and enables the patient to be restored immediately, so the limitations of patient customized crowns that make it impossible to immediately restore a single tooth may be overcome.

Second, it has the effect of easily applying a prefabricated crown in a method of classifying and tabulating a plurality of prefabricated crowns by type, size, material and color by a table system, manufacturing a plurality of prefabricated crowns corresponding to the table, and then selecting a prefabricated crown suitable for the patient from the table.

Third, the scope of treatment may be extended to low-income patients around the world who have no choice but to maintain the oral environment in a state of permanently missing teeth by either not receiving timely treatment or giving up treatment due to economic and time problems even though dental crown treatment is required in a timely manner, so it may be widely used as a material for immediate prosthetic restoration of a single tooth.

Fourth, aesthetics and biocompatibility are excellent because ceramic is a main component unlike conventional metal and resin crowns, in particular, aesthetics is excellent and biocompatibility is high because zirconium oxide ceramics stabilized with yttrium oxide having high strength and toughness and/or zirconium oxide ceramics containing a colorant stabilized with yttrium oxide are used as raw materials, so it may be widely applied to the industrialization of medical materials.

### [Description of Drawings]

Figure 1 is a flowchart showing a method for manufacturing a prefabricated crown according to one embodiment of the present invention.
Figure 2 is a block diagram schematically showing the structure of a table system for classifying and tabulating the data of the prefabricated crowns shown in Figure 1 by type, size, material and color.
Figure 3 is a schematic view of 48 teeth treatable by the present invention and a prefabricated crown immediately prosthetic restored to this individual single tooth.
Figure 4 is a schematic diagram specifying the three-dimensional dimensions of the prefabricated crown for sub-gingival and the prefabricated crown for supra-gingival manufactured by the present invention.
Figure 5 is a schematic diagram specifying the three-dimensional dimensions of the actual crown and the try-in crown manufactured by the present invention.
Figure 6 is a drawing showing an example of a table describing the type, size, color and material of the prefabricated crown processed by the table system of Figure 2.

### [Best Mode]

Hereinafter, a prefabricated crown for dental prosthetic restoration and a method for manufacturing the same according to one embodiment of the present invention will be described in detail with reference to the accompanying drawings.

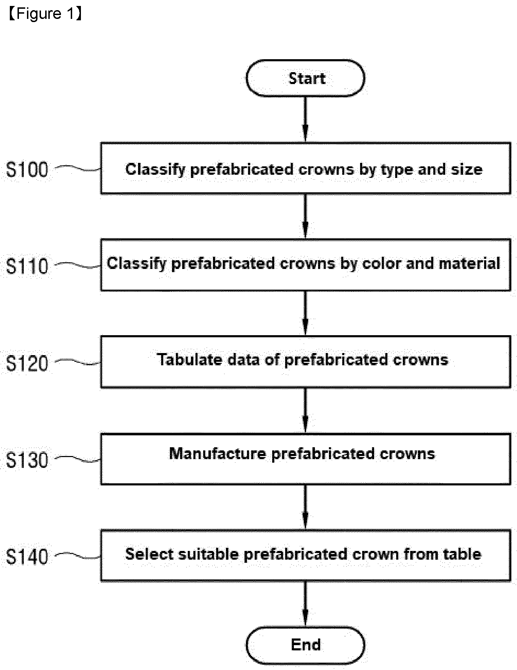

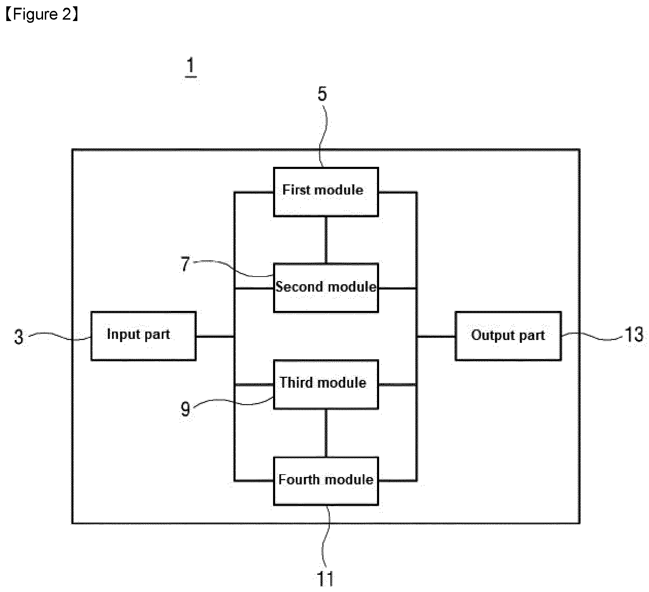

As shown in Figures 1 to 6, the method for manufacturing a prefabricated crown proposed by the present invention is a technology for classifying, calculating and tabulating data on the type, size, material and color of the prefabricated crowns by the table system 1, and manufacturing a plurality of prefabricated crowns corresponding to the table, and then selecting a prefabricated crown suitable for a patient from the table.

In this case, the table system 1 refers to a device for processing data using an input and output device and a central processing unit, such as a PC.

This method for manufacturing a prefabricated crown
relates to a method for manufacturing a prefabricated crown by processing and tabulating prefabricated crowns for sub-gingival and supra-gingival and actual and try-in crowns by type, size, material and color by the table system 1.

More specifically, the method for manufacturing a prefabricated crown comprises a first step S100 of classifying a plurality of prefabricated crowns by type and size by a first module 5 of the table system 1; a second step S110 of classifying the plurality of prefabricated crowns by color and material by a second module 7 of the table system 1; a third step S120 of tabulating the type, size, color and material data of the prefabricated crowns classified in the first and second steps S100, S110 by a third module 9 of the table system 1, and assigning an identification code to each data; a fourth step S130 of manufacturing the tabulated plurality of prefabricated crowns; and a fifth step S140 of inputting the measured type, size, color and material data of patient's tooth into a fourth module 11 of the table system 1 to select a prefabricated crown corresponding thereto.

The method for manufacturing a prefabricated crown described above will be described in more detail as follows.

In the first step S100, a plurality of prefabricated crowns are classified by type and size.

Specifically, the first step S100 comprises classifying the prefabricated crowns by type and size by the first module 5 by inputting whether the prefabricated crowns are sub-gingival or supra-gingival, and actual or try-in through the input part 3 of the table system 1 and inputting the three-dimensional size.

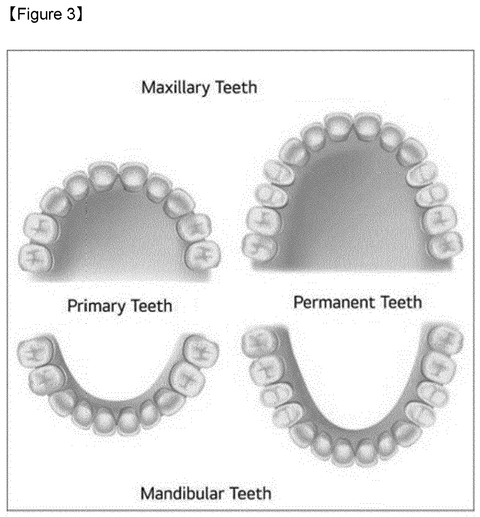

That is, as shown in Figure 3, for 48 teeth (28 permanent teeth + 20 primary teeth), a total of 192 single-tooth prefabricated crowns including 96 actual crowns for sub-gingival and supra-gingival and 96 try-in crowns for sub-gingival and supra-gingival for immediate prosthetic restoration are classified by type and size.

In this classification process, the types and sizes of a plurality of prefabricated crowns, for example, 192 prefabricated crowns, are input by the input part 3 of the table system 1, and the input type and size data are processed by the first module 5.

At this time, the types of the prefabricated crowns are classified into whether they are sub-gingival or supra-gingival, or actual or try-in.

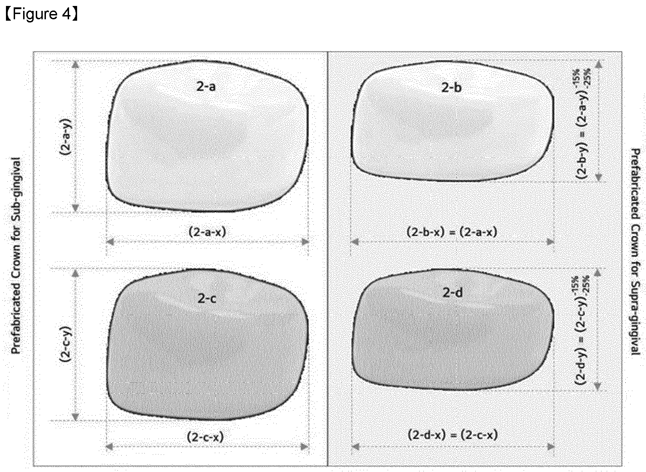

In addition, in Figure 4, the three-dimensional dimension 2-b-y between the gum part and the occlusal part of the actual crown for supra-gingival 2-b (prefabricated crown for supra-gingival) is less than the three-dimensional dimension 2-a-y between the gum part and the occlusal part of the actual crown for sub-gingival 2-a (prefabricated crown for sub-gingival), and substantially, it means that the three-dimensional dimension 2-b-y between the gum part and the occlusal part of the actual crown for supra-gingival 2-b is -15% to -25% smaller than the three-dimensional dimension 2-a-y between the gum part and the occlusal part of the actual crown for sub-gingival 2-a.

That is, if the 3D dimension between the gum part and the occlusal part of the prefabricated crown for supra-gingival is the same as the 3D dimension between the gum part and the occlusal part of the prefabricated crown for sub-gingival, the prefabricated crown for sub-gingival is actualized in the supra-gingival treatment case, resulting in excessive tooth removal. Therefore, a three-dimensional dimension design and tolerance setting of a prefabricated crown optimized for supra-gingival treatment are required.

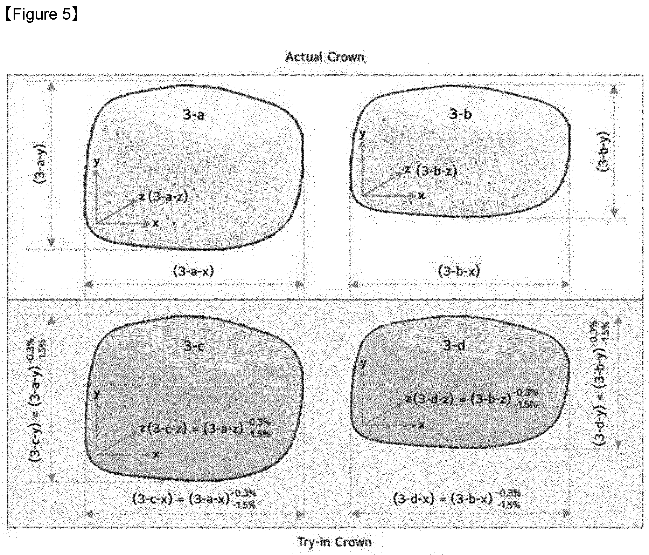

Within the invention, as shown in Figure 5, the three-dimensional dimensions 3-c-x, 3-c-y, 3-c-z of the try-in crown for sub-gingival 3-c and the three-dimensional dimensions 3-d-x, 3-d-y, 3-d-z of the try-in crown for supra-gingival 3-d are less than the three-dimensional dimensions 3-a-x, b3-a-y, 3-a-z of the actual crown for sub-gingival 3-a and the three-dimensional dimensions 3-b-x, 3-b-y, 3-b-z of the actual crown for supra-gingival 3-b, respectively, and substantially, it means that the three-dimensional dimensions 3-c-x, 3-c-y, 3-c-z of the try-in crown for sub-gingival 3-c and the three-dimensional dimensions 3-d-x, 3-d-y, 3-d-z of the try-in crown for supra-gingival 3-d is -0.3% to -1.5% smaller than the three-dimensional dimensions 3-a-x, 3-a-y, 3-a-z of the actual crown for sub-gingival 3-a and the three-dimensional dimensions 3-b-x, 3-b-y, 3-b-z of the actual crown for supra-gingival 3-b, respectively.

That is, in the case of try-in and actualization with an actual crown without a try-in crown, the retention force with the inner surface of the crown is rapidly reduced due to contamination by saliva and blood during the try-in process for selecting the size of the crown and securing the cementing position, resulting in a debonding phenomenon.

In addition, in the case of try-in with a try-in crown without a constant three-dimensional dimension standard between the try-in crown and the actual crown, the three-dimensional fitting is inappropriate when actualized with the actual crown. Therefore, a three-dimensional dimension design and tolerance setting of a try-in crown optimized for three-dimensional fitting are required.

In the second step S110, the prefabricated crowns are classified by color and material.

Specifically, the second step S110 comprises inputting the material and color of the prefabricated crowns through the input part 3 of the table system 1 and classifying the prefabricated crowns by material and color by the second module 7.

That is, as an example, color and material data for 192 prefabricated crowns are input through the input part 3 of the table system 1, and the input color and material data are processed and classified by the second module 7.

More specifically, since a prefabricated crown is formed of various materials, it includes, for example, sintered ceramics, particularly oxide ceramics, and preferably, it may include a zirconium oxide ceramic stabilized with yttrium oxide or a zirconium oxide ceramic containing a colorant stabilized with yttrium oxide.

In this case, the "oxide ceramic" means a dental ceramic of a certain composition in order to distinguish it from a "silicate ceramic".

The oxide ceramic uses a simple oxide of metal as a matrix, and as an example, this oxide ceramic may use zirconium oxide (ZrO₂, zirconia), aluminum oxide (Al₂O₃), titanium dioxide (TiO₂) or magnesium oxide (MgO) as a matrix.

Meanwhile, the oxide ceramic may be modified by adding another metal oxide, for example, modified zirconium oxide. Zirconium oxide may be stabilized at lower temperatures due to high temperature modification. In this case, the metal oxide suitable for the modification may include, for example, calcium oxide (CaO), magnesium oxide (MgO), cerium oxide (CeO, CeO₃), or particularly yttrium oxide (Y₂O₃, yttria).

In addition, for the modified zirconium oxide, fully stabilized zirconia (FSZ) and partially stabilized zirconia (PSZ) are distinguished from each other, and the partially stabilized zirconia refers to tetragonal zirconia polycrystal (TZP).

As an example, yttria-stabilized tetragonal zirconia polycrystal (Y-TZP) refers to the corresponding stabilized zirconia based on yttrium oxide.

In addition, the zirconium oxide ceramic stabilized with yttrium oxide may include various types, for example, 3Y-TZP (3 mol% yttria doped tetragonal zirconia polycrystal), 4Y-TZP (4 mol% yttria doped tetragonal zirconia polycrystal), and 5Y-TZP (5 mol% yttria doped tetragonal zirconia polycrystal) containing 3 mol%, 4 mol% and/or 5 mol% of yttrium oxide, respectively.

In addition, of course, the zirconium oxide ceramic stabilized with yttrium oxide may contain a small amount of hafnium oxide (HfO₂) and aluminum oxide (Al₂O₃) as a natural companion of zirconium oxide (ZrO₂), for example, up to 5% hafnium oxide and up to 0.5% aluminum oxide.

Meanwhile, the zirconium oxide ceramic stabilized with yttrium oxide may include a colorant. As a specific example, the colorants that may contain such fluorescent materials may include at least one component selected from erbium (Er₂O₃), terbium oxide (Tb₄O₇), thulium oxide (Tm₂O₃), red iron oxide (Fe₂O₃), yellow iron oxide (Fe₂O₃), molybdenum oxide (MnO₃)b, manganese dioxide (MnO₂), cerium oxide (CeO₂), barium oxide (BaO), vanadium pentoxide (V₂O₅), vanadium trioxide (V₂O₃) and cobalt oxide (CO₃O₄), and its content may be within the range of up to 0.5%.

Examples of such zirconium oxide ceramics stabilized with yttrium oxide or zirconium oxide ceramics containing a colorant stabilized with yttrium oxide are shown in Tables 1 to 3 below.

**[Table 1]**

| Component Name | Amount(%) |
|---|---|
| Yttrium oxide (Y₂O₃) | >4.5 to ≤6.0 |
| Hafnium oxide (HfO₂) | ≤5.0 |
| Aluminum oxide (Al₂O₃) | ≤0.5 |
| Other oxide + colorant | ≤0.5 |
| Zirconium oxide + hafnium oxide + yttrium oxide (ZrO₂+HfO₂+Y₂O₃) | ≥99.0 |

**[Table 2]**

| Component Name | Amount(%) |
|---|---|
| Yttrium oxide (Y₂O₃) | >6.0 to ≤7.5 |
| Hafnium oxide (HfO₂) | ≤5.0 |
| Aluminum oxide (Al₂O₃) | ≤0.5 |
| Other oxide + colorant | ≤0.5 |
| Zirconium oxide + hafnium oxide + yttrium oxide(ZrO₂+HfO₂+Y₂O₃) | ≥99.0 |

**[Table 3]**

| Component Name | Amount(%) |
|---|---|
| Yttrium oxide (Y₂O₃) | >6.0 to ≤7.5 |
| Hafnium oxide (HfO₂) | ≤5.0 |
| Aluminum oxide (Al₂O₃) | ≤0.5 |
| Other oxide + colorant | ≤0.5 |
| Zirconium oxide + hafnium oxide + yttrium oxide (ZrO₂+HfO₂+Y₂O₃) | ≥99.0 |

In addition, as other examples of the oxide ceramic in the present invention, a mixed oxide ceramic based on aluminum oxide (Al₂O₃) and zirconium oxide (ZrO₂) as main components may be included. Typically, they may be mixed oxide ceramics comprising 80% to 90% aluminum oxide with the balance zirconium oxide, or about 80% zirconium oxide with the balance aluminum oxide. Such mixed oxide ceramics are, for example, referred to as zirconia toughened alumina (ZTA) or alumina toughened zirconia (ATZ).

On the other hand, the first and second steps S100, S110 are performed as described above, and then the third step S120 is performed, and in the third step S120, the data classified in the first and second steps S100, S110 is tabulated by the third module 9 of the table system 1, and an identification code is assigned to each data.

Specifically, it comprises matching and tabulating the type, size, color and material data of the prefabricated crowns classified by the first module 5 and the second module 7 with each other by the third module 9, and may be a step of tabulating each identification code by assigning identification codes by classifying the types of the prefabricated crowns into supra-gingival or sub-gingival, or actual or try-in; assigning identification codes by aligning the sizes of the prefabricated crowns so that each prefabricated crown may be distinguished from each other; assigning identification codes so that a plurality of ceramics may be distinguished from each other in the materials of the prefabricated crowns and expressing the content of the ceramics to be distinguished in the form of numbers in the identification codes; and assigning identification codes so that the colorants that may include a plurality of fluorescent materials may be distinguished from each other in the colors of the prefabricated crowns and expressing the content of the colorants to be distinguished in the form of numbers in the identification codes.

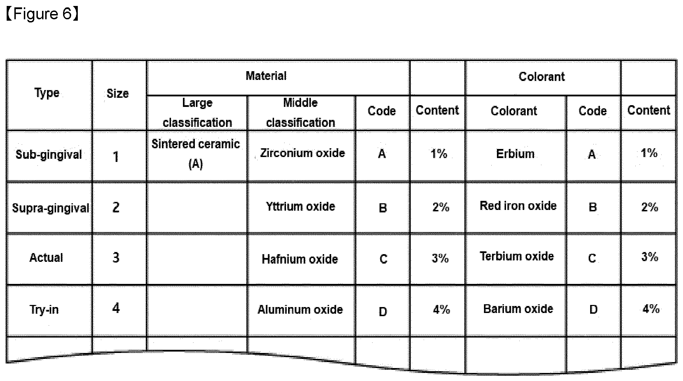

That is, as shown in Figure 6, an identification code is assigned to 192 prefabricated crowns by size and, for example, the code is assigned as serial number, and it is assigned as 1 for the first prefabricated crown and as 2 for the second prefabricated crown.

In addition, an identification code is assigned by color and material and, for example, as a large classification, the code is assigned as A for sintered ceramics and as B for general ceramics. In addition, as a middle classification, the code is assigned as A for zirconium oxides, as B for yttrium oxides, as C for hafnium oxides, and as D for aluminum oxides.

In this case, the content of each component of the middle classification may be expressed as a number. For example, for 5% yttrium oxide, the code is assigned as B05.

In addition, as a small classification, the code is assigned for colorants, and the code is set as A for erbium and as B for red iron oxide, and the content of each colorant is expressed as numbers. That is, 0.5% erbium is expressed as A005.

These processes may be tabulated by processing the size data, color data, and material data input by the input part 3 by the operation part. Therefore, when selecting a prefabricated crown suitable for the patient, it may be easily selected by this table.

As such, in the third step S120, the size, color and material of the prefabricated crowns may be matched and tabulated by code.

On the other hand, in the fourth step S130, each tabulated prefabricated crown is manufactured. Such prefabricated crowns may be manufactured by various processing methods and may be by, for example, an injection molding method, a subtractive manufacturing method, or an additive manufacturing method.

During the injection process of prefabricated crowns, they are manufactured using zirconium oxide ceramics stabilized with yttrium oxide or zirconium oxide ceramics containing a colorant stabilized with yttrium oxide, and are manufactured by mixing ceramics and polymers, injection molding, debinding, and sintering them, and then polishing them.

Specifically, in a specific embodiment of the present invention, in the fourth step S130, the prefabricated crowns are manufactured by any one of an injection molding method, a subtractive manufacturing and an additive manufacturing method, and the injection molding may comprise molding a zirconium oxide ceramic stabilized with yttrium oxide or a zirconium oxide ceramic containing a colorant stabilized with yttrium oxide, and the steps of: mixing 70 to 85% by weight of ceramic and 15 to 30% by weight of at least one polymer selected from the group consisting of paraffin wax, low-density polyethylene and ethylene vinyl acetate by heating at 150 to 180°C; injection molding the heated and mixed mixture at a temperature of 150 to 200°C and a pressure of 30 to 40 MPa for 30 to 100 seconds to manufacturing a injection-molded body of a prefabricated crown; debinding the injection molded body by a first heat treatment at 25 to 250°C for 20 to 30 hours, followed by a second heat treatment at 200 to 550°C for 20 to 30 hours; sintering the debinded injection molded body by heat treatment at 1,400 to 1,600°C for 1 to 5 hours; and polishing the outer surface of the heat-treated sintered body to impart gloss.

As a material for the prefabricated crown that may reproduce a thin margin thickness of 0.20 to 0.50 mm, satisfy both aesthetics and strength properties at the same time, and have excellent biocompatibility to maintain the dental restoration shape in the treated state even if it is used permanently, it may be preferable to use, for example, a zirconium oxide ceramic stabilized with yttrium oxide or a zirconium oxide ceramic containing a colorant stabilized with yttrium oxide.

The polymer is mixed to lower viscosity and impart moldability during injection molding, and may include at least one polymer selected from paraffin wax, low density polyethylene, and ethylene vinyl acetate.

In the above and below description, it will be understood that "polymer" is not limited to a composition consisting of only a polymer, but includes a polymer composition including various additives, a processing aid or a glidant such as stearic acid added in conventional injection molding.

First, 15 to 30% by weight of a polymer is added to 70 to 85% by weight of ceramic, and a plasticizer may be added to facilitate molding if necessary, and the mixture of ceramic and polymer is mixed by heating at 150 to 180°C.

The ceramic consists of fine and uniformly pulverized ceramic powder, and the heating is to prevent defects by stably forming a crown shape during injection molding while softening the polymer so as to be uniformly mixed with the ceramic.

Next, an injection-molded body of a preformed crown is manufactured by injection molding the heated and mixed mixture of ceramic and polymer.

Injection molding is performed under various temperature and pressure conditions, for example, at a temperature of 150 to 200°C and a pressure of 30 to 40 MPa for 30 to 100 seconds.

At this time, if the injection molding temperature is less than 150°C, the flowability of the mixture of ceramic and polymer is low, so molding may be difficult, and a shape such as a weld bead may occur, which may bring about product defects, and if it is more than 200°C, debinding of the polymer may occur during mixing of the ceramic and the polymer, which may create voids in the injection-molded body.

As such, in the present invention, since the prefabricated crown may be manufactured through the injection molding method, it may be easily applied when mass production is required.

The injection-molded body of the prefabricated crown manufactured in the injection molding process is brittle, so there is a risk of surface damage during processing, and the polymer component present in the injection-molded body acts as a factor that deteriorates the mechanical properties of the prefabricated crown by generating voids or microcracks in the heat treatment process to be described later, and thus, for the purpose of preventing them, a debinding process is performed to lower the brittleness and generate ductility of the injection-molded body and to remove the polymer component of the injection-molded body.

The debinding process may be performed by a first heat treatment of the injection-molded body at 25 to 250°C for 20 to 30 hours, followed by a second heat treatment at 200 to 550°C for 20 to 30 hours, and more specifically, may be carried out as a process of a first heating at 100 to 250°C for 20 to 30 hours and then a second heat treatment at 250 to 550°C for 20 to 30 hours in order to burn and remove the polymer component remaining in the injection-molded body.

As such, as the temperature is raised and debinded step by step, thermal stress on the injection-molded body is suppressed and deformation of the injection-molded body is minimized accordingly, and the polymer component present on the surface of the injection-molded body is first debinded, and then the internal polymer component is sequentially debinded, thereby suppressing the occurrence of structural non-uniformity of the injection-molded body according to the debinding.

If the temperature of the debinding process is less than the above temperature and time range, there is a risk of damage to the prefabricated crown due to the occurrence of voids or cracks in the heat treatment process because the polymer remains in the injection-molded body, and if it is more than the above temperature and time range or stepwise heating is not performed, deformation or breakage of the molded body is likely to occur.

Next, the ceramic is sintered by a sintering process of heat-treating the debinded injection-molded body at 1400 to 1600°C for 1 to 5 hours. More specifically, the debinded body is heat treated at 1500 to 1600°C for 1 to 2 hours, but the heat treatment temperature is preferably the above temperature in consideration of diffusion of ceramic particles and necking between particles, and if the heat treatment temperature is less than the above range, the properties of the sintered body may be deteriorated due to incomplete sintering, and if it is more than the above range, mechanical properties may be reduced due to excessive grain growth.

The polymer is composed of organic compounds containing carbon as a main component, and the organic compound is decomposed in the above temperature range of the debinding process, or combined with oxygen and discharged to the outside of the injection-molded body to remove most thereof, but the carbon component remaining after decomposition of the organic compound remains inside the injection-molded body, and this carbon component may increase the brittleness of the injection-molded body, causing surface damage during barrel processing.

The temperature increase rate to the heat treatment temperature is preferably 0.5 to 5.0°C/min, and if the temperature increase rate is less than 0.5°C/min, the temperature increase time takes a long time to lower productivity, and if it is more than 5.0°C/min, thermal stress due to a sudden temperature change may act on the injection-molded body.

The debinding process and the heat treatment process may be carried out continuously, that is, the efficiency of the process may be secured and energy may be saved by directly raising the temperature without cooling the second-heated injection body and heat-treating it in the debinding process.

However, if the injection-molded body of the injection-molded prefabricated crown is heated to a high temperature in the debinding process and heat treatment process, density unevenness and thin margin thickness of the injection-molded body due to uneven molding pressure that may occur during the injection molding process may cause deformation of the injection-molded body of the prefabricated crown.

As a method for preventing this problem, the debinding process and the sintering process may be discontinuously performed, that is, deformation of the prefabricated crown can be prevented by placing the debinded injection-molded body cooled to room temperature on a sagger having the outer surface shape of a plurality of prefabricated crowns and heat-treating them at a high temperature again.

That is, the structural non-uniformity inside the injection-molded body is resolved through the process of heating, cooling and heating the injection-molded body, and the crown deformation phenomenon may be solved by using the sagger having the outer surface shape of the prefabricated crowns, so that there is an advantage in that the prefabricated crown having a thin margin thickness may be easily sintered.

A barrel processing is performed to impart gloss and remove fine burrs by polishing the outer surface of the sintered body sintered by the sintering process. The barrel processing process may be performed using a barrel polisher or the like.

On the other hand, the prefabricated crown of the present invention may also be manufactured by subtractive manufacturing.

In the case of subtractive manufacturing, it is manufactured by subtractive manufacturing a ceramic blank comprising a zirconium oxide stabilized with yttrium oxide and/or a zirconium oxide containing a colorant stabilized with yttrium oxide, debinding and sintering, and then polishing them in the same manner as in the injection molding process.

Specifically, in another specific embodiment of the present invention, in the fourth step S130, the prefabricated crowns are manufactured by any one of an injection molding method, a subtractive manufacturing and an additive manufacturing method, and the subtractive manufacturing may comprise subtractive manufacturing a ceramic blank comprising a zirconium oxide stabilized with yttrium oxide and/or a zirconium oxide containing a colorant stabilized with yttrium oxide, and the steps of: clamping a ceramic blank comprising a zirconium oxide stabilized with yttrium oxide and/or a zirconium oxide containing a colorant stabilized with yttrium oxide to a subtractive manufacturing machine; importing a file of an anatomically designed prefabricated shape for each single tooth into the subtractive manufacturing machine to subtractive manufacture the clamped blank into a prefabricated crown, and processing it into a workpiece of a prefabricated crown; debinding the workpiece by a first heat treatment at 25 to 600°C for 1 to 10 hours, followed by a second heat treatment at 600 to 1000°C for 1 to 10 hours; sintering the debinded workpiece by heat treatment at 1,500 to 1,600°C for 1 to 5 hours; and polishing the outer surface of the heat-treated sintered body to impart gloss, and wherein the blank has a disk shape and is processed into a plurality of crown workpieces as a blank for single tooth.

On the one hand, the ceramic blank may be an unsintered, i.e., not sintered ceramic material or (only) partially sintered, i.e., not finally sintered or densely sintered ceramic material. These materials are still, to varying degrees, porous, but may be more easily subtractive manufactured than the finally sintered (densely sintered) ceramic material.

Specifically, in order to enable the prefabricated crown for immediate prosthetic restoration of a single tooth according to the present invention to reproduce a thin margin thickness of 0.20 to 0.50 mm of the prefabricated crown, satisfy both aesthetics and strength properties at the same time, and have excellent biocompatibility to maintain the dental restoration shape in the treated state even if it is used permanently, the ceramic blank is preferably a zirconium oxide ceramic stabilized with yttrium oxide and/or a zirconium oxide ceramic containing a colorant stabilized with yttrium oxide.

It is preferable to perform subtractive manufacturing using the bur of which the diameter for reproducing the thin margin thickness of the prefabricated crown is 2.5 mm or less, particularly 1 mm or less, particularly preferably 0.6 mm or less.

The blank may preferably be in the form of a disc with a diameter of more than 20 mm, in particular more than 50 mm, and particularly preferably more than 80 mm, and a height of 10 to 20 mm, and a single tooth blank as the blank is preferably processed into a workpiece of a plurality of crowns, preferably more than 5 and in particular more than 10 crowns.

In addition, the debinding step consists of a process of a first heat treatment at 25 to 600°C for 1 to 10 hours, followed by a second heat treatment at 600 to 1,000°C for 1 to 10 hours in order to burn and remove the organic material remaining on the workpiece. The sintering step comprises heat-treating the debinded body from which the binder is removed at 1,500 to 1,600°C for 1 to 5 hours, and the debinding step and the sintering step are preferably performed continuously in one furnace.

Of course, the heat treatment temperature and time are not limited to the above ranges and may be appropriately changed according to the manufacturing environment.

On the other hand, the prefabricated crown of the present invention may also be manufactured by additive manufacturing.

In the case of additive manufacturing, it is manufactured by additive manufacturing a ceramic composition comprising a zirconium oxide stabilized with yttrium oxide or a zirconium oxide containing a colorant stabilized with yttrium oxide, debinding and sintering, and then polishing them in the same manner as in the injection molding process.

Specifically, in another specific embodiment of the present invention, in the fourth step S130, the prefabricated crowns are manufactured by one of an injection molding method, a subtractive manufacturing method, and an additive manufacturing method, and the additive manufacturing may comprise additive manufacturing a ceramic composition comprising a zirconium oxide stabilized with yttrium oxide or a zirconium oxide containing a colorant stabilized with yttrium oxide, and the steps of: injecting the ceramic composition into the additive manufacturing machine; importing a file of an anatomically designed prefabricated shape for each single tooth into the additive manufacturing machine to additive manufacture the injected composition into a prefabricated crown, and forming it into a work piece of a prefabricated crown; debinding the work piece a first heat treatment at 25 to 600°C for 1 to 10 hours, followed by a secondary heat treatment at 600 to 1000°C for 1 to 10 hours; sintering the debinded work piece by heat treatment at 1500 to 1,600°C for 1 to 5 hours; and polishing the outer surface of the heat-treated sintered work piece to impart gloss, and wherein the ceramic composition is made by mixing a thermosetting resin comprising 5 to 10% by weight of an epoxy resin, 1 to 10% by weight of maleic acid, 1 to 10% by weight of 3,6,9-trioxadecanoic acid and 1 to 10% by weight of an amine compound based on 100% by weight of a liquid silicone resin, and modified zirconium oxide powders stabilized with yttrium oxide or zirconium oxide powders containing a colorant stabilized with yttrium oxide in a weight ratio of 0.8 to 1.2:1, and wherein the modified zirconium oxide powders stabilized with yttrium oxide or zirconium oxide powders containing a colorant stabilized with yttrium oxide are obtained by mixing a composition comprising 5 to 15% by weight of a silane compound, 1 to 10% by weight of an ethyl acrylate monomer and 1 to 10% by weight of a solvent at a temperature of 120 to 250°C based on 100% by weight of unmodified zirconium oxide powders stabilized with yttrium oxide or zirconium oxide powders containing a colorant stabilized with yttrium oxide, and wherein the work piece may be formed from a a single tooth work piece into a plurality of crown formed bodies.

This prefabricated crown to reproduce a thin margin thickness of 0.20 to 0.50 mm of the prefabricated crown, satisfy both aesthetics and strength properties at the same time, and have excellent biocompatibility to maintain the dental restoration shape in the treated state even if it is used permanently, it is preferable to use an additive product of a ceramic composition comprising a zirconium oxide stabilized with yttrium oxide or a zirconium oxide containing a colorant stabilized with yttrium oxide.

The ceramic composition comprising a zirconium oxide stabilized with yttrium oxide or a zirconium oxide containing a colorant stabilized with yttrium oxide specifically comprises a ceramic powder and a thermosetting resin, wherein the ceramic powder and the thermosetting resin may be mixed, for example, in a weight ratio of 1:0.8 to 1.2, and the viscosity may be exemplified by 1,000 to 100,000 cps.

Of course, the above % by weight and viscosity may be appropriately changed depending on the manufacturing environment.

On the one hand, the ceramic powders may be modified ceramic powders, specifically, modified zirconium oxide ceramic powders stabilized with yttrium oxide and/or zirconium oxide ceramic powders containing a colorant stabilized with yttrium oxide.

The modified ceramic powders may be made, for example, by mixing a composition comprising 5 to 15% by weight of a silane compound, 1 to 10% by weight of an ethyl acrylate monomer and 1 to 10% by weight of a solvent based on 100% by weight of the ceramic powders at a temperature of 120 to 250°C.

The % by weight and the temperature range may be appropriately changed depending on the manufacturing environment.

The silane compound is for improving bonding strength with the thermosetting resin, has an epoxy group at one end and a silane group at the other end, and includes 2-(3,4-epoxycyclohexyl) ethyltrimethoxy silane, aminopropyl triethoxy silane, and aminoethyl aminopropyl trimethoxy silane.

The solvent may include any one of isopropyl glycol, methyl ethyl ketone, dimethylformamide, and ethanol.

The thermosetting resin may include any one of phenol resin (PF, phenol-formaldehyde resin), urea resin (UF, urea-formaldehyde resin), melamine resin (MF, melamine-formaldehyde resin), unsaturated polyester resin (UP), epoxy Resin (EP), furan resin, silicone resin, alkyd resin, polyamide resin (PA), casein resin, and diallyl phthalate resin (DAP).

In particular, a silicone resin excellent in biocompatibility, heat resistance, tensile strength and bonding strength may be preferable. Therefore, an embodiment in which the thermosetting resin is a liquid silicone resin will be described below.

The thermosetting resin may include 5 to 10% by weight of an epoxy resin, 1 to 10% by weight of maleic acid, 1 to 10% by weight of 3,6,9-trioxadecanoic acid, and 1 to 10% by weight of an amine compound based on 100% by weight of a liquid silicone.

In this case, the amine compound may be triethanolamine or triisopropanolamine.

The thermosetting resin may further include a catalyst or a plasticizer. The catalyst may include chloroplatinic acid or dimethyl phosphate, and its content may be 5 to 15% by weight based on 100% by weight of the thermosetting resin. The plasticizer may include butyl benzyl phthalate, and its content may be 0.1 to 1.0% by weight based on 100% by weight of the thermosetting resin.

In addition, the thermosetting resin is a component for crosslinking and curing upon heating, and an example thereof may include an organic peroxide such as benzoyl peroxide, and its content may be 0.1 to 5.0% by weight based on 100% by weight of the thermosetting resin.

In a specific embodiment, as the composition for additive manufacturing having the above composition, a composition comprising 5 to 10% by weight of an epoxy resin, 1 to 10% by weight of maleic acid, 1 to 10% by weight of 3,6,9-trioxadecanoic acid, 1 to 10% by weight of an amine compound, 5 to 15% by weight of a catalyst, 0.1 to 1.0% by weight of a plasticizer and 0.1 to 5.0% by weight of a curing agent based on 100% by weight of the liquid silicone resin is prepared, and then mixed with a zirconium oxide ceramic stabilized with yttrium oxide and/or a zirconium oxide ceramic powder containing a colorant stabilized with yttrium oxide in a weight ratio of 1:1, and defoamed to prepare a slurry composition, thereby manufacturing a additive product according to the above-described method.

The % by weight and the weight ratio are not limited to the above range and may be appropriately changed according to the manufacturing environment.

On the one hand, the ceramic powders may be preferably modified zirconium oxide ceramics stabilized with yttrium oxide and/or zirconium oxide ceramic powders containing a colorant stabilized with yttrium oxide as described above, and the effect exhibited by such modified ceramic powders may be confirmed from the following examples.

In the additive manufacturing method, in Example 1, a composition was prepared as follows:
A thermosetting resin is prepared by mixing 100% by weight of a liquid silicone resin with 12% by weight of chloroplatinic acid and 3% by weight of benzoyl peroxide at a temperature of 150°C.

This thermosetting resin is mixed with 100% by weight of zirconium oxide ceramics stabilized with yttrium oxide having an average particle size of 50 µm and/or zirconium oxide ceramic powders containing a colorant stabilized with the yttrium oxide and defoamed to prepare a 3D additive composition in a slurry state.

On the other hand, in the additive manufacturing method, in Example 2, a composition was prepared as follows:
A composition for a 3D additive printer is prepared in the same manner as in Example 1, except that modified zirconium oxide ceramics stabilized with yttrium oxide and/or zirconium oxide ceramic powders containing a colorant stabilized with yttrium oxide are used instead of zirconium oxide ceramics stabilized with yttrium oxide and/or zirconium oxide ceramic powders containing a colorant stabilized with yttrium oxide in Example 1.

In this case, 100% by weight of the modified zirconium oxide ceramics stabilized with yttrium oxide and/or zirconium oxide ceramic powders containing a colorant stabilized with yttrium oxide were mixed with 5% by weight of 2-(3,4-epoxycyclohexyl) ethyltrimethoxy silane, 5% by weight of ethyl acrylate monomer, 5% by weight of acrylic acid and 10% by weight of isopropyl glycol at a temperature of 150°C.

Finally, in the additive manufacturing method, in Example 3, a composition was prepared as follows:
A composition for a 3D additive printer is prepared in the same manner as in Example 2, except that the liquid silicone resin is modified in the thermosetting resin of Example 2. In this case, the modified thermosetting resin consists of 5% by weight of epoxy resin, 5% by weight of maleic acid, 5% by weight of 3,6,9-trioxadecanoic acid and 3% by weight of triisopropanol amine based on 100% by weight of the liquid silicone resin.

The ceramic compositions for additive manufacturing of the above-described Examples 1 to 3 are put into an additive manufacturing equipment and heat-cured at 200°C to prepare molded bodies of 50*50*50 mm (width*length*height), and the compressive strength and wear resistance of each molded body are measured and shown in Table 4 below. In this case, the wear resistance is the result of measuring the wear loss per 100 revolutions with the equipment specified in KSM 6080.

**[Table 4]**

| | Example 1 | Example 2 | Example 3 |
|---|---|---|---|
| Compressive strength (MPa) | 63 | 73 | 108 |
| Wear resistance (mg) | 89 | 34 | 12 |

As can be seen in Table 4 above, it can be seen that the inclusion of modified zirconium oxide ceramics stabilized with yttrium oxide and/or zirconium oxide ceramic powders containing a colorant stabilized with yttrium oxide as in Examples 2 and 3 as a ceramic composition for additive manufacturing contribute to the improvement of compressive strength and wear resistance, and furthermore, it can be seen that when a modified material also are used as a thermosetting resin, it shows the most desirable result in improving the compressive strength and wear resistance in the additive product.

The molded body to be additive manufactured by the composition is preferably additive manufactured to a plurality of crowns, preferably more than 5, particularly more than 10 crowns as a molded body for a single tooth, and it is preferable to 3D mold and cure using a synthetic resin applicable to a thermosetting 3D printer and a zirconium oxide stabilized with yttrium oxide and/or a zirconium oxide ceramic powder containing a colorant stabilized with yttrium oxide so that the disadvantages of 3D printers of the stereolithography apparatus (SLA) method using conventional photocurable resins to additive manufacture the crown molded body may be compensated.

In addition, the debinding step preferably consists of a process of a first heat treatment at 25 to 600°C for 1 to 10 hours and then a second heat treatment at 600 to 1000°C for 1 to 10 hours in order to burn and remove the polymer component remaining in the molded body, the sintering step preferably comprises heat-treating the debinded body from which the polymer is removed at 1500 to 1600°C for 1 to 5 hours, and the debinding step and the sintering step are preferably performed continuously in one furnace.

Finally, in the method for manufacturing a prefabricated crown according to the present invention, after the fourth step S130 of manufacturing a prefabricated crown by a injection molding method, a subtractive manufacturing method, or an additive manufacturing method as described above is completed, the fifth step S140 of selecting a prefabricated crown suitable for a patient among the manufactured prefabricated crowns is performed.

Specifically, the fifth step S140 is a step of inputting the measured type, size, color and material data of patient's tooth into the fourth module 11 of the table system 1 to select a prefabricated crown corresponding thereto.

In the fifth step S140, first, the type and size of a tooth for prosthetic restoration applied to the patient is measured, and a prefabricated crown corresponding to the measured data is selected from the table.

First, it is established whether the types of the prefabricated crowns for prosthetic restoration are sub-gingival or supra-gingival, or actual or try-in.

Then, the size of the dentition to which the prefabricated crown in the patient's mouth is applied is measured. That is, the size of the part to be restored, such as the maxillary teeth, permanent teeth, primary teeth, and mandibular teeth, is measured.

After the type and size of the prefabricated crown are measured in this way, the corresponding data is input through the fourth module 11 of the table system 1 to calculate the corresponding prefabricated crown from the table.

For example, if the prefabricated crown required by the patient is a permanent tooth, for sub-gingival and actual, and the size is (X,Y,Z) mm, the prefabricated crown corresponding to the data is selected from the table.

In addition, the material and color of the prefabricated crown required by the patient are determined, and the corresponding prefabricated crown is selected from the table.

For example, if the prefabricated crown material is 5% yttrium oxide and the colorant is 0.5% erbium, a prefabricated crown corresponding to the code B05A005 is selected from the table, and as another example, if the prefabricated crown material is 5% yttrium oxide and the colorant is 0.25% erbium and 0.25% terbium oxide, a prefabricated crown corresponding to the code B05A0025C0025 is selected from the table.

In this way, after a prefabricated crown suitable for the patient is selected from the table, the corresponding prefabricated crown is operated on the patient among the pre-manufactured prefabricated crowns.

### [Industrial Applicability]

The present invention is useful in processing a prefabricated crown providing a prosthetic restoration to patients quickly by tabulating the variables of the type, three-dimensional dimension, color and material of prefabricated crowns for sub-gingival and supra-gingival, and actual and try-in crowns, and manufacturing a prefabricated crown corresponding to the table in advance.

## Claims

1. A method for manufacturing a plurality of prefabricated crowns including try-in and actual crowns by processing and tabulating
the prefabricated crowns for sub-gingival and supra-gingival, and
the actual and try-in crowns by type, size, material and color by a table system (1),
the method comprising:
a first step (S100) of classifying a plurality of prefabricated crowns by type and size by a first module (5) of the table system (1);
a second step (S110) of classifying the plurality of prefabricated crowns by color and material by a second module (7) of the table system (1);
a third step (S120) of tabulating the type, size, color and material data of the prefabricated crowns classified in the first and second steps (S100, S110) by a third module (9) of the table system (1), and assigning an identification code to each data;
a fourth step (S130) of manufacturing a plurality of prefabricated crowns including the try-in and actual crowns corresponding to the table system; and
a fifth step (S140) of inputting the measured type, size, color and material data of patient's tooth into a fourth module (11) of the table system (1) to select a prefabricated crown corresponding thereto,
wherein the prefabricated crown has three-dimensional dimensions (X, Y, Z) meaning the length in the X-axis, Y-axis and Z-axis directions, and the three-dimensional dimensions (3-c-x, 3-c-y, 3-c-z ; 3-d-x, 3-d-y, 3-d-z) of the try-in crown are smaller than the three-dimensional dimensions (3-a-x, 3-a-y, 3-a-z; 3-b-x, 3-b-y, 3-b-z) of the actual crown, and
the three-dimensional dimensions (3-c-x, 3-c-y, 3-c-z ; 3-d-x, 3-d-y, 3-d-z) of the try-in crown are smaller by -1.5% to -0.3% based on the three-dimensional dimensions (3-a-x, b3-a-y, 3-a-z; 3-b-x, 3-b-y, 3-b-z) of the actual crown.

2. The method for manufacturing a prefabricated crown according to claim 1, wherein in the fourth step (S130), the prefabricated crowns are manufactured by any one of an injection molding method, a subtractive manufacturing method, and an additive manufacturing method, and the injection molding method comprises molding a zirconium oxide ceramic stabilized with yttrium oxide or a zirconium oxide ceramic containing a colorant stabilized with yttrium oxide, and the steps of:
mixing 70 to 85% by weight of ceramic and 15 to 30% by weight of at least one polymer selected from the group consisting of paraffin wax, low-density polyethylene and ethylene vinyl acetate by heating at 150 to 180°C;
injection molding the heated and mixed mixture at a temperature of 150 to 200°C and a pressure of 30 to 40 MPa for 30 to 100 seconds to manufacturing a injection-molded body of a prefabricated crown;
debinding the injection molded body by a first heat treatment at 25 to 250°C for 20 to 30 hours, followed by a second heat treatment at 200 to 550°C for 20 to 30 hours;
sintering the debinded injection molded body by heat treatment at 1,400 to 1,600°C for 1 to 5 hours; and
polishing the outer surface of the heat-treated sintered body to impart gloss.

3. The method for manufacturing a prefabricated crown according to claim 1, wherein in the fourth step (S130), the prefabricated crowns are manufactured by any one of an injection molding method, a subtractive manufacturing method, and an additive manufacturing method, and the subtractive manufacturing method comprises subtractive manufacturing a ceramic blank comprising a zirconium oxide stabilized with yttrium oxide and/or a zirconium oxide containing a colorant stabilized with yttrium oxide, and the steps of:
clamping a ceramic blank comprising a zirconium oxide stabilized with yttrium oxide and/or a zirconium oxide containing a colorant stabilized with yttrium oxide to a subtractive manufacturing machine;
importing a file of an anatomically designed prefabricated shape for each single tooth into the subtractive manufacturing machine to subtractive manufacture the clamped blank into a prefabricated crown, and processing it into a workpiece of a prefabricated crown;
debinding the workpiece by a first heat treatment at 25 to 600°C for 1 to 10 hours, followed by a second heat treatment at 600 to 1000°C for 1 to 10 hours;
sintering the debinded workpiece by heat treatment at 1,500 to 1,600°C for 1 to 5 hours; and
polishing the outer surface of the heat-treated sintered body to impart gloss, and
wherein the blank has a disk shape and is processed into a plurality of crown workpieces as a blank for single tooth.

4. The method for manufacturing a prefabricated crown according to claim 1, wherein in the fourth step (S130), the prefabricated crowns are manufactured by one of an injection molding method, a subtractive manufacturing method, and an additive manufacturing method, and the additive manufacturing method comprises additive manufacturing a ceramic composition comprising a zirconium oxide stabilized with yttrium oxide or a zirconium oxide containing a colorant stabilized with yttrium oxide, and the steps of:
injecting the ceramic composition into the additive manufacturing machine;
importing a file of an anatomically designed prefabricated shape for each single tooth into the additive manufacturing machine to additive manufacture the injected composition into a prefabricated crown, and forming it into a work piece of a prefabricated crown;
debinding the work piece by a first heat treatment at 25 to 600°C for 1 to 10 hours, followed by a second heat treatment at 600 to 1000°C for 1 to 10 hours;
sintering the debinded work pieceby heat treatment at 1500 to 1,600°C for 1 to 5 hours; and
polishing the outer surface of the heat-treated sintered work piece to impart gloss, and
wherein the ceramic composition is made by mixing a thermosetting resin comprising 5 to 10% by weight of an epoxy resin, 1 to 10% by weight of maleic acid, 1 to 10% by weight of 3,6,9-trioxadecanoic acid and 1 to 10% by weight of an amine compound based on 100% by weight of a liquid silicone resin, and modified zirconium oxide powders stabilized with yttrium oxide or zirconium oxide powders containing a colorant stabilized with yttrium oxide in a weight ratio of 0.8 to 1.2:1, wherein the modified zirconium oxide powders stabilized with yttrium oxide or zirconium oxide powders containing a colorant stabilized with yttrium oxide are obtained by mixing a composition comprising 5 to 15% by weight of a silane compound, 1 to 10% by weight of an ethyl acrylate monomer and 1 to 10% by weight of a solvent at a temperature of 120 to 250°C based on 100% by weight of unmodified zirconium oxide powder stabilized with yttrium oxide or zirconium oxide powder containing a colorant stabilized with yttrium oxide, and
wherein the work piece is formed from a single tooth work piece into a plurality of crown formed bodies.

5. The method for manufacturing a prefabricated crown according to claim 1, wherein in the first step (S100), the prefabricated crowns are classified by type and size by the first module (5) by inputting whether the prefabricated crowns are sub-gingival or supra-gingival, and actual or try-in through an input part (3) of the table system (1) and inputting the three-dimensional size.

6. The method for manufacturing a prefabricated crown according to claim 1, wherein the second step (S110) is a step of inputting the material and color of the prefabricated crowns through the input part (3) of the table system (1) and classifying the prefabricated crowns by material and color by the second module (7).

7. The method for manufacturing a prefabricated crown according to claim 1, wherein the third step (S120) comprises matching and tabulating the type, size, color and material data of the prefabricated crowns classified by the first module (5) and the second module (7) with each other by the third module (9), and is a step of tabulating each identification code by assigning identification codes by classifying the types of the prefabricated crowns into supra-gingival or sub-gingival, or actual or try-in; assigning identification codes by aligning the sizes of the prefabricated crowns so that each prefabricated crown is distinguished from each other; assigning identification codes so that a plurality of ceramics may be distinguished from each other in the materials of the prefabricated crowns and expressing the content of the ceramics to be distinguished in the form of numbers in the identification codes; and assigning identification codes so that the colorants that comprise a plurality of fluorescent materials are distinguished from each other in the colors of the prefabricated crowns and expressing the content of the colorants to be distinguished in the form of numbers in the identification codes.

8. Prefabricated crowns obtained by the method of any one of claims 1 to 7, the prefabricated crowns comprising a try-in crown and an actual crown, wherein the prefabricated crowns have three-dimensional dimensions (X, Y, Z) meaning the length in the X-axis, Y-axis and Z-axis directions, and the three-dimensional dimensions of the try-in crown is smaller than the three-dimensional dimensions of the actual crown, and the three-dimensional dimensions of the try-in crown is small by -1.5% to -0.3% based on the three-dimensional dimensions of the actual crown.

9. The prefabricated crowns according to claim 8, wherein each of the try-in crown and/or actual crown comprises a sub-gingival crown and a supra-gingival crown.

10. The prefabricated crowns according to claim 8, wherein the prefabricated crowns comprise a zirconium oxide ceramic stabilized with yttrium oxide or a zirconium oxide ceramic containing a colorant stabilized with yttrium oxide.

11. The prefabricated crowns according to claim 10, wherein the colorant comprises one or more selected from the group consisting of erbium (Er₂O₃), terbium oxide (Tb₄O₇), thulium oxide (Tm₂O₃), red iron oxide (Fe₂O₃), yellow iron oxide (Fe₂O₃), molybdenum oxide (Mn0₃), manganese dioxide (MnO₂), cerium oxide (CeO₂), barium oxide (BaO), vanadium pentoxide (V₂O₅), vanadium trioxide (V₂O₃) and cobalt oxide (CO₃O₄).

12. The prefabricated crowns according to claim 10, wherein the colorant is included in an amount of not more than 0.5% by weight based on the total weight of the zirconium oxide ceramic containing a colorant stabilized with yttrium oxide.

## Patentansprüche

1. Verfahren zur Herstellung einer Vielzahl von vorgefertigten Kronen mit Einprobe- und tatsächlichen Kronen durch Verarbeiten und Tabellieren
der vorgefertigten Kronen für subgingivale und supragingivale Anwendungen und
der tatsächlichen und Einprobekronen nach Typ, Größe, Material und Farbe mittels eines Tischsystems (1),
welches Verfahren Folgendes umfasst:
einen ersten Schritt (S100) des Klassifizierens einer Vielzahl von vorgefertigten Kronen nach Typ und Größe mittels eines ersten Moduls (5) des Tischsystems (1);
einen zweiten Schritt (S110) des Klassifizierens der Vielzahl von vorgefertigten Kronen nach Farbe und Material mittels eines zweiten Moduls (7) des Tischsystems (1);
einen dritten Schritt (S120) des Tabellierens der Typ-, Größen-, Farb- und Materialdaten der vorgefertigten Kronen, die in den ersten und zweiten Schritten (S100, S110) klassifiziert werden, mittels eines dritten Moduls (9) des Tischsystems (1) und des Zuweisens eines Identifikationscodes zu jeder der Daten;
einen vierten Schritt (S130) des Herstellens einer Vielzahl von vorgefertigten Kronen mit den Einprobe- und tatsächlichen Kronen entsprechend dem Tischsystem; und
einen fünften Schritt (S140) des Eingebens der gemessenen Typ-, Größen-, Farb- und Materialdaten des Patientenzahns in ein viertes Modul (11) des Tischsystems (1), um eine diesem entsprechende vorgefertigte Krone auszuwählen,
wobei die vorgefertigte Krone dreidimensionale Abmessungen (X, Y, Z) aufweist, die die Länge in den Richtungen der X-Achse, Y-Achse und Z-Achse bedeuten, und die dreidimensionalen Abmessungen (3-c-x, 3-c-y, 3-c-z; 3-d-x, 3-d-y, 3-d-z) der Einprobekrone kleiner als die dreidimensionalen Abmessungen (3-a-x, b3-a-y, 3-a-z; 3-b-x, 3-b-y, 3-b-z) der tatsächlichen Krone sind, und
wobei die dreidimensionalen Abmessungen (3-c-x, 3-c-y, 3-c-z; 3-d-x, 3-d-y, 3-d-z) der Einprobekrone um -1,5% bis -0,3% bezogen auf die dreidimensionalen Abmessungen (3-a-x, b3-a-y, 3-a-z; 3-b-x, 3-b-y, 3-b-z) der tatsächlichen Krone kleiner sind.

2. Verfahren zur Herstellung einer vorgefertigten Krone nach Anspruch 1, wobei im vierten Schritt (S130) die vorgefertigten Kronen durch ein beliebiges von einem Spritzgussverfahren, einem subtraktiven Herstellungsverfahren und einem additiven Herstellungsverfahren hergestellt werden, und wobei das Spritzgussverfahren das Formen einer mit Yttriumoxid stabilisierten Zirkonoxidkeramik oder einer Zirkonoxidkeramik, die einen mit Yttriumoxid stabilisierten Farbstoff enthält, und die folgenden Schritte umfasst:
Mischen von 70 bis 85 Gew.-% Keramik und 15 bis 30 Gew.-% mindestens eines Polymers, das aus der Gruppe bestehend aus Paraffinwachs, Polyethylen niedriger Dichte und Ethylenvinylacetat ausgewählt ist, durch Erhitzen auf 150 bis 180°C;
Spritzgießen der erhitzten und gemischten Mischung bei einer Temperatur von 150 bis 200°C und einem Druck von 30 bis 40 MPa für 30 bis 100 Sekunden zur Herstellung eines Spritzgusskörpers einer vorgefertigten Krone;
Entbindern des Spritzgusskörpers durch eine erste Wärmebehandlung bei 25 bis 250°C für 20 bis 30 Stunden, gefolgt von einer zweiten Wärmebehandlung bei 200 bis 550°C für 20 bis 30 Stunden;
Sintern des entbinderten Spritzgusskörpers durch Wärmebehandlung bei 1400 bis 1600°C für 1 bis 5 Stunden; und
Polieren der Außenfläche des wärmebehandelten Sinterkörpers, um ihm Glanz zu verleihen.

3. Verfahren zur Herstellung einer vorgefertigten Krone nach Anspruch 1, wobei im vierten Schritt (S130) die vorgefertigten Kronen durch ein beliebiges von einem Spritzgussverfahren, einem subtraktiven Herstellungsverfahren und einem additiven Herstellungsverfahren hergestellt werden, und wobei das subtraktive Herstellungsverfahren das subtraktive Herstellen eines Keramikrohlings, der ein mit Yttriumoxid stabilisiertes Zirkonoxid und/oder ein Zirkonoxid, das einen mit Yttriumoxid stabilisierten Farbstoff enthält, beinhaltet, und die folgenden Schritte umfasst:
Einspannen eines Keramikrohlings, der ein mit Yttriumoxid stabilisiertes Zirkonoxid und/oder ein Zirkonoxid, das einen mit Yttriumoxid stabilisierten Farbstoff enthält, beinhaltet, in eine subtraktive Herstellungsmaschine;
Importieren einer Datei einer anatomisch gestalteten vorgefertigten Form für jeden einzelnen Zahn in die subtraktive Herstellungsmaschine, um den eingespannten Rohling zu einer vorgefertigten Krone subtraktiv herzustellen und Verarbeiten dieses Rohlings zu einem Werkstück einer vorgefertigten Krone;
Entbindern des Werkstücks durch eine erste Wärmebehandlung bei 25 bis 600°C für 1 bis 10 Stunden, gefolgt von einer zweiten Wärmebehandlung bei 600 bis 1000°C für 1 bis 10 Stunden;
Sintern des entbinderten Werkstücks durch Wärmebehandlung bei 1500 bis 1600°C für 1 bis 5 Stunden; und Polieren der Außenfläche des wärmebehandelten Sinterkörpers, um ihm Glanz zu verleihen, und
wobei der Rohling eine Scheibenform aufweist und zu einer Vielzahl von Kronenwerkstücken als Rohling für einen einzelnen Zahn verarbeitet wird.

4. Verfahren zur Herstellung einer vorgefertigten Krone nach Anspruch 1, wobei im vierten Schritt (S130) die vorgefertigten Kronen durch eines von einem Spritzgussverfahren, einem subtraktiven Herstellungsverfahren und einem additiven Herstellungsverfahren hergestellt werden, und wobei das additive Herstellungsverfahren das additive Herstellen einer Keramikzusammensetzung, die ein mit Yttriumoxid stabilisiertes Zirkonoxid oder ein Zirkonoxid, das einen mit Yttriumoxid stabilisierten Farbstoff enthält, beinhaltet, und die folgenden Schritte umfasst:
Einspritzen der Keramikzusammensetzung in die additive Herstellungsmaschine;
Importieren einer Datei einer anatomisch gestalteten vorgefertigten Form für jeden einzelnen Zahn in die additive Herstellungsmaschine, um die eingespritzte Zusammensetzung zu einer vorgefertigten Krone additiv herzustellen, und Formen dieser Zusammensetzung zu einem Werkstück einer vorgefertigten Krone;
Entbindern des Werkstücks durch eine erste Wärmebehandlung bei 25 bis 600°C für 1 bis 10 Stunden, gefolgt von einer zweiten Wärmebehandlung bei 600 bis 1000°C für 1 bis 10 Stunden;
Sintern des entbinderten Werkstücks durch Wärmebehandlung bei 1500 bis 1600°C für 1 bis 5 Stunden; und Polieren der Außenfläche des wärmebehandelten gesinterten Werkstücks, um ihm Glanz zu verleihen, und
wobei die Keramikzusammensetzung durch Mischen eines duroplastischen Harzes bestehend aus 5 bis 10 Gew.-% eines Epoxidharzes, 1 bis 10 Gew.-% Maleinsäure, 1 bis 10 Gew.-% 3,6, 9-Trioxadecansäure und 1 bis 10 Gew.-% einer Aminverbindung, bezogen auf 100 Gew.-% eines flüssigen Silikonharzes, und modifizierter Zirkonoxidpulver, die mit Yttriumoxid stabilisiert sind, oder Zirkonoxidpulver, die einen mit Yttriumoxid stabilisierten Farbstoff enthalten, in einem Gewichtsverhältnis von 0,8 bis 1,2:1 hergestellt wird, wobei die modifizierten Zirkonoxidpulver, die mit Yttriumoxid stabilisiert sind, oder Zirkonoxidpulver, die einen mit Yttriumoxid stabilisierten Farbstoff enthalten, durch Mischen einer Zusammensetzung erhalten werden, die 5 bis 15 Gew.-% einer Silanverbindung, 1 bis 10 Gew.-% eines Ethylacrylatmonomers und 1 bis 10 Gew.-% eines Lösungsmittels bei einer Temperatur von 120 bis 250°C, bezogen auf 100 Gew.-% unmodifiziertes Zirkonoxidpulver, das mit Yttriumoxid stabilisiert ist, oder Zirkonoxidpulver, das einen mit Yttriumoxid stabilisierten Farbstoff enthält, und
wobei das Werkstück aus einem einzelnen Zahnwerkstück zu einer Vielzahl von Kronenformkörpern geformt wird.

5. Verfahren zur Herstellung einer vorgefertigten Krone nach Anspruch 1, wobei im ersten Schritt (S100) die vorgefertigten Kronen nach Typ und Größe mittels des ersten Moduls (5) durch Eingeben, ob es sich bei den vorgefertigten Kronen um subgingivale oder supragingivale und um tatsächliche oder Einprobekronen handelt, über einen Eingangsteil (3) des Tischsystems (1) und Eingeben der dreidimensionalen Größe klassifiziert werden.

6. Verfahren zur Herstellung einer vorgefertigten Krone nach Anspruch 1, wobei der zweite Schritt (S110) ein Schritt des Eingebens des Materials und der Farbe der vorgefertigten Kronen über den Eingangsteil (3) des Tischsystems (1) und des Klassifizierens der vorgefertigten Kronen nach Material und Farbe mittels des zweiten Moduls (7) ist.

7. Verfahren zur Herstellung einer vorgefertigten Krone nach Anspruch 1, wobei der dritte Schritt (S120) das Abgleichen und Tabellieren der Typ-, Größen-, Farb- und Materialdaten der durch das erste Modul (5) und das zweite Modul (7) klassifizierten vorgefertigten Kronen mittels des dritten Moduls (9) umfasst und ein Schritt des Tabellierens jedes Identifizierungscodes durch Zuweisen von Identifizierungscodes mittels Klassifizieren der Typen der vorgefertigten Kronen in supragingival oder subgingival oder tatsächlich oder zur Einprobe ist; Zuweisen von Identifizierungscodes durch Anpassen der Größen der vorgefertigten Kronen, so dass jede vorgefertigte Krone voneinander unterschieden wird; Zuweisen von Identifikationscodes, so dass eine Vielzahl von Keramiken hinsichtlich der Materialien der vorgefertigten Kronen voneinander unterschieden werden kann und Ausdrücken des Inhalts der zu unterscheidenden Keramiken in Form von Zahlen in den Identifikationscodes; und Zuweisen von Identifikationscodes, so dass die Farbstoffe, die eine Vielzahl von fluoreszierenden Materialien umfassen, hinsichtlich der Farben der vorgefertigten Kronen voneinander unterschieden werden und Ausdrücken des Inhalts der zu unterscheidenden Farbstoffe in Form von Zahlen in den Identifikationscodes.

8. Vorgefertigte Kronen, die mit dem Verfahren nach einem der Ansprüche 1 bis 7 erhalten werden, mit einer Einprobekrone und einer tatsächlichen Krone, wobei die vorgefertigten Kronen dreidimensionale Abmessungen (X, Y, Z) aufweisen, die die Länge in den Richtungen der X-Achse, der Y-Achse und der Z-Achse bedeuten, und wobei die dreidimensionalen Abmessungen der Einprobekrone kleiner als die dreidimensionalen Abmessungen der tatsächlichen Krone sind, während die dreidimensionalen Abmessungen der Einprobekrone um -1,5% bis -0,3% bezogen auf die dreidimensionalen Abmessungen der tatsächlichen Krone kleiner sind.

9. Vorgefertigte Kronen nach Anspruch 8, wobei jede der Einprobekronen und/oder tatsächlichen Kronen eine subgingivale Krone und eine supragingivale Krone umfasst.

10. Vorgefertigte Kronen nach Anspruch 8, wobei die vorgefertigten Kronen eine mit Yttriumoxid stabilisierte Zirkonoxidkeramik oder eine Zirkonoxidkeramik, die einen mit Yttriumoxid stabilisierten Farbstoff enthält, umfassen.

11. Vorgefertigte Kronen nach Anspruch 10, wobei der Farbstoff eines oder mehrere davon umfasst, ausgewählt aus der Gruppe bestehend aus Erbium (Er₂O₃), Terbiumoxid (Tb₄O₇), Thuliumoxid (Tm₂O₃), rotem Eisenoxid (Fe₂O₃), gelbem Eisenoxid (Fe₂O₃), Molybdänoxid (MnO₃), Mangandioxid (MnO₂), Ceroxid (CeO₂), Bariumoxid (BaO), Vanadiumpentoxid (V₂O₅), Vanadiumtrioxid (V₂O₃) und Kobaltoxid (CO₃O₄).

12. Vorgefertigte Kronen nach Anspruch 10, wobei der Farbstoff in einer Menge von nicht mehr als 0,5 Gew.-%, bezogen auf das Gesamtgewicht der Zirkonoxidkeramik, die einen mit Yttriumoxid stabilisierten Farbstoff enthält, beinhaltet ist.

## Revendications

1. Procédé de fabrication d'une pluralité de couronnes préfabriquées comprenant des couronnes d'essayage et des couronnes réelles, consistant à traiter et tabuler les couronnes préfabriquées pour sous-gingivales et supra-gingivales, et les couronnes réelles et d'essayage par type, taille, matériau et couleur par un système de table (1),
le procédé comprenant :
une première étape (S100) consistant à classer une pluralité de couronnes préfabriquées par type et taille par un premier module (5) du système de table (1) ;
une deuxième étape (S110) consistant à classer la pluralité de couronnes préfabriquées par couleur et matériau par un deuxième module (7) du système de table (1) ;
une troisième étape (S120) consistant à tabuler des données relatives au type, à la taille, à la couleur et au matériau des couronnes préfabriquées classées aux première et deuxième étapes (S100, S110) par un troisième module (9) du système de table (1), et à attribuer un code d'identification à chaque donnée ;
une quatrième étape (S130) consistant à fabriquer une pluralité de couronnes préfabriquées comprenant les couronnes d'essayage et les couronnes réelles correspondant au système de table ; et
une cinquième étape (S140) consistant à saisir les données mesurées relatives au type, à la taille, à la couleur et au matériau de dents d'un patient dans un quatrième module (11) du système de table (1) pour sélectionner une couronne préfabriquée correspondant à celles-ci,
dans lequel la couronne préfabriquée a des dimensions tridimensionnelles (X, Y, Z), soient la longueur dans des directions d'un axe X, d'un axe Y et d'un axe Z, et les dimensions tridimensionnelles (3-c-x, 3-c-y, 3-c-z ; 3-d-x, 3-d-y, 3-d-z) de la couronne d'essayage sont inférieures aux dimensions tridimensionnelles (3-a-x, b3-a-y, 3-a-z ; 3-b-x, 3-b-y, 3-b-z) de la couronne réelle, et
les dimensions tridimensionnelles (3-c-x, 3-c-y, 3-c-z ; 3-d-x, 3-d-y, 3-d-z) de la couronne d'essayage sont inférieures de -1,5 % à -0,3 % sur la base des dimensions tridimensionnelles (3-a-x, b3-a-y, 3-a-z ; 3-b-x, 3-b-y, 3-b-z) de la couronne réelle.

2. Procédé de fabrication d'une couronne préfabriquée selon la revendication 1, dans lequel à la quatrième étape (S130), les couronnes préfabriquées sont fabriquées par l'une quelconque parmi une méthode de moulage par injection, une méthode de fabrication soustractive et une méthode de fabrication additive, et la méthode de moulage par injection consiste à mouler une céramique d'oxyde de zirconium stabilisée avec l'oxyde d'yttrium ou une céramique d'oxyde de zirconium contenant un colorant stabilisé avec l'oxyde d'yttrium, et comprend les étapes suivantes :
mélanger 70 à 85 % en poids de céramique et 15 à 30 % en poids d'au moins un polymère choisi dans le groupe comprenant la cire de paraffine, le polyéthylène à basse densité et l'éthylène-acétate de vinyle en chauffant à 150 à 180°C ;
mouler par injection le mélange chauffé et mélangé à une température de 150 à 200°C et une pression de 30 à 40 MPa pendant 30 à 100 secondes pour fabriquer un corps moulé par injection d'une couronne préfabriquée ;
retirer le corps moulé par injection par un premier traitement thermique à 25 à 250°C pendant 20 à 30 heures, suivi d'un deuxième traitement thermique à 200 à 550°C pendant 20 à 30 heures ;
fritter le corps moulé par injection par un traitement thermique à 1400 à 1600 °C pendant 1 à 5 heures ; et
polir la surface extérieure du corps fritté et traité thermiquement pour donner une brillance.

3. Procédé de fabrication d'une couronne préfabriquée selon la revendication 1, dans lequel à la quatrième étape (S130), les couronnes préfabriquées sont fabriquées par l'une quelconque parmi une méthode de moulage par injection, une méthode de fabrication soustractive et une méthode de fabrication additive, et la méthode de fabrication soustractive consiste à fabriquer de manière soustractive une ébauche en céramique comprenant un oxyde de zirconium stabilisé avec l'oxyde d'yttrium et/ou un oxyde de zirconium contenant un colorant stabilisé avec l'oxyde d'yttrium, et comprend les étapes suivantes :
serrer une ébauche en céramique comprenant un oxyde de zirconium stabilisé avec l'oxyde d'yttrium et/ou un oxyde de zirconium contenant un colorant stabilisé avec l'oxyde d'yttrium à une machine de fabrication soustractive ;
introduire une lime d'une forme préfabriquée établie anatomiquement pour chaque dent dans la machine de fabrication soustractive pour fabriquer de manière soustractive l'ébauche serrée dans une couronne préfabriquée, et transformer la en une pièce à usiner d'une couronne préfabriquée ;
retirer la pièce à usiner par un premier traitement thermique à 25 à 600°C pendant 1 à 10 heures, suivi d'un deuxième traitement thermique à 600 à 1000°C pendant 1 à 10 heures ;
fritter la pièce à usiner retirée par un traitement thermique à 1 500 à 1600 °C pendant 1 à 5 heures ; et
polir la surface extérieure du corps fritté et traité thermiquement pour donner une brillance, et
dans lequel l'ébauche a une forme de disque et est transformée en une pluralité de pièce à usiner de couronne comme ébauche pour une seule dent.

4. Procédé de fabrication d'une couronne préfabriquée selon la revendication 1, dans lequel à la quatrième étape (S 130), les couronnes préfabriquées sont fabriquées par l'une parmi une méthode de moulage par injection, une méthode de fabrication soustractive et une méthode de fabrication additive, et la méthode de fabrication additive consiste à fabriquer de manière additive une composition céramique comprenant un oxyde de zirconium stabilisé avec l'oxyde d'yttrium ou un oxyde de zirconium contenant un colorant stabilisé avec l'oxyde d'yttrium, et comprend les étapes suivantes :
injecter la composition céramique dans la machine de fabrication additive ;
introduire une lime d'une forme préfabriquée établie anatomiquement pour chaque dent dans la machine de fabrication additive pour fabriquer de manière additive la composition céramique dans une couronne préfabriquée, et transformer la en une pièce à usiner d'une couronne préfabriquée ;
retirer la pièce à usiner par un premier traitement thermique à 25 à 600°C pendant 1 à 10 heures, suivi d'un deuxième traitement thermique à 600 à 1000°C pendant 1 à 10 heures ;
fritter la pièce à usiner retirée par un traitement thermique à 1500 à 1600 °C pendant 1 à 5 heures ; et
polir la surface extérieure de la pièce à usiner fritté et traité thermiquement pour donner une brillance, et
dans lequel la composition céramique est obtenue en mélangeant une résine thermodurcissable comprenant 5 à 10 % en poids de résine époxy, 1 à 10 % en poids d'acide maléique, 1 à 10 % en poids d'acide 3,6,9-trioxadecanoïque et 1 à 10 % en poids d'un composé amine sur la base de 100 % en poids d'une résine de silicone liquide, et de l'oxyde de zirconium en poudre modifié et stabilisé avec l'oxyde d'yttrium ou de l'oxyde de zirconium en poudre contenant un colorant stabilisé avec l'oxyde d'yttrium dans un rapport en poids de 0,8 à 1,2 : 1, dans lequel l'oxyde de zirconium en poudre modifié et stabilisé avec l'oxyde d'yttrium ou l'oxyde de zirconium en poudre contenant un colorant stabilisé avec l'oxyde d'yttrium est obtenu en mélangeant une composition comprenant 5 à 15 % en poids d'un composé de silane, 1 à 10 % en poids d'un monomère d'acrylate d'éthyle et 1 à 10 % en poids d'un solvant à une température de 120 à 250 °C sur la base de 100 % en poids d'oxyde de zirconium en poudre non modifiée stabilisé avec l'oxyde d'yttrium ou l'oxyde de zirconium en poudre contenant un colorant stabilisé avec l'oxyde d'yttrium, et
dans lequel la pièce à usiner est formée à partir d'une pièce à usiner de seule dent en une pluralité de corps formés de couronne.

5. Procédé de fabrication d'une couronne préfabriquée selon la revendication 1, dans lequel, à la première étape (S100), les couronnes préfabriquées sont classées par type et taille par le premier module (5) en saisissant si les couronnes préfabriquées sont sous-gingivales ou supra-gingivales, et réelles ou d'essayage à l'aide d'une portion de saisie (3) du système de table (1) et en saisissant la taille tridimensionnelle.

6. Procédé de fabrication d'une couronne préfabriquée selon la revendication 1, dans lequel la deuxième étape (S110) est une étape consistant à saisir le matériau et la couleur des couronnes préfabriquées par la portion de saisie (3) du système de table (1) et classer les couronnes préfabriquées par matériau et couleur par le deuxième module (7).

7. Procédé de fabrication d'une couronne préfabriquée selon la revendication 1, dans lequel
la troisième étape (S120) consiste à faire correspondre ou tabuler les données relatives au type, à la taille, à la couleur et au matériau des couronnes préfabriquées classées par le premier module (5) et le deuxième module (7) les unes avec les autres par le troisième module (9), et est une étape de tabulation de chaque code d'identification consistant à attribuer des codes d'identification en classant les types des couronnes préfabriquées en supra-gingivaux ou sous-gingivaux, ou réelles ou d'essayage ; attribuer des codes d'identification en alignant les dimensions des couronnes préfabriquées de sorte que chaque couronne préfabriquée soit distinguée l'une de l'autre ; attribuer des codes d'identification de sorte qu'une pluralité de céramiques soient distinguées les unes des autres dans les matériaux des couronnes préfabriquées et exprimer le contenu des céramiques à distinguer sous forme de numéros dans les codes d'identification ; et attribuer des codes d'identification de sorte que les colorants comprenant une pluralité de matériaux fluorescents soient distingués les uns des autres par les couleurs des couronnes préfabriquées et exprimer le contenu des colorants à distinguer sous forme de numéros dans les codes d'identification.

8. Couronnes préfabriquées obtenues par le procédé selon l'une quelconque des revendications 1 à 7, les couronnes préfabriquées comprenant une couronne d'essayage et une couronne réelle, dans lesquelles les couronnes préfabriquées ont des dimensions tridimensionnelles (X, Y, Z), soient la longueur dans des directions d'un axe X, d'un axe Y et d'un axe Z, et les dimensions tridimensionnelles de la couronne d'essayage sont inférieures aux dimensions tridimensionnelles de la couronne réelle, et les dimensions tridimensionnelles de la couronne d'essayage sont inférieures de -1,5 % à -0,3 % sur la base des dimensions tridimensionnelles de la couronne réelle.

9. Couronnes préfabriquées selon la revendication 8, dans lesquelles chacune de la couronne d'essayage et/ou de la couronne réelle comprend une couronne sous-gingivale et une couronne supra-gingivale.

10. Couronnes préfabriquées selon la revendication 8, dans lesquelles les couronnes préfabriquées comprennent une céramique d'oxyde de zirconium stabilisée avec l'oxyde d'yttrium ou une céramique d'oxyde de zirconium contenant un colorant stabilisé avec l'oxyde d'yttrium.

11. Couronnes préfabriquées selon la revendication 10, dans lesquelles le colorant comprend un ou plusieurs choisis dans un groupe comprenant l'erbium (Er₂O₃), l'oxyde de terbium (Tb₄O₇), l'oxyde de thulium (Tm₂O₃), l'oxyde de fer rouge (Fe₂O₃), l'oxyde de fer jaune (Fe₂O₃), l'oxyde de molybdène (MnO₃), le dioxyde de manganèse (MnO₂), l'oxyde de cérium (CeO₂), l'oxyde de baryum (BaO), le pentoxyde de vanadium (V₂O₅), le trioxyde de vanadium (V₂O₃) et l'oxyde de cobalt (CO₃O₄).

12. Couronnes préfabriquées selon la revendication 10, dans lesquelles le colorant représente une quantité ne dépassant pas 0,5 % en poids sur la base du poids total de la céramique d'oxyde de zirconium contenant un colorant stabilisé avec l'oxyde d'yttrium.
